# EUROPEAN PATENT APPLICATION

(11) **EP 2 554 268 A1**
(43) Date of publication of application: **06.02.2013**
(21) Application number: 11762225.8
(22) Date of filing: 28.03.2011
(51) Int. Cl.: B01J 38/68, B01J 27/199, B01J 27/28, C07B 61/00, C07C 51/235, C07C 57/055

(54) **PROCESS FOR PRODUCTION OF CATALYST FROM RECOVERED CATALYST**

(30) Priority: 29.03.2010 JP 2010076232
(71) Applicant: Nippon Kayaku Kabushiki Kaisha, Tokyo 102-8172 (JP)
(72) Inventor: KURAKAMI, Tatsuhiko, Sanyoonoda-shi Yamaguchi 757-8686 (JP); IIJIMA, Takayuki, Sanyoonoda-shi Yamaguchi 757-8686 (JP); KOBAYASHI, Tomoaki, Sanyoonoda-shi Yamaguchi 757-8686 (JP); KOJIMA, Toshitake, Takasaki-shi Gunma 370-1208 (JP)
(74) Representative: Gille Hrabal
(86) International application number: PCT/JP2011/001820
(87) International publication number: WO 2011/121976

(57) **Abstract**

The present invention relates to a method for manufacturing a regenerated catalyst from a recovered catalyst from manufacturing process or a used catalyst of a heteropoly acid-based catalyst containing molybdenum, phosphorus, vanadium or copper as an essential component by the below-described Process a to Process f:
Process a: a solution A is prepared by mixing a substance M with an aqueous solvent and removing a component insoluble in the solvent;
Process b: the molar quantity of at least one component of molybdenum, phosphorus, vanadium or copper contained in the solution A is measured;
Process c: an aqueous hydrogen peroxide solution is added to the solution A to obtain a solution B;
Process d: the difference between the content obtained in Process b and the theoretical value of a required element is determined and the shortage amount of the catalyst component is added to the solution B to prepare a solution C;
Process e: the solution C is dried to prepare catalyst granules D; and
Process f: the catalyst granules D are molded and subsequently calcined to prepare a molded catalyst E,

the manufacturing method is also simple, and the obtained said regenerated catalyst has performance equivalent to an unused target catalyst, leading to a large advantage that it can be used as it is in combination with an unused target catalyst.

## Description

### Technical Field

The present invention relates to a method for manufacturing a regenerated heteropoly acid-based catalyst from a recovered heteropoly acid-based catalyst, where the method for manufacturing a catalyst is characterized by using a spent catalyst as a raw material or as a part of raw materials to manufacture a regenerated catalyst.

For example, techniques for recycling a heteropoly acid-based catalyst for manufacturing methacrylic acid are described in, for example, Patent Literature 1 and Patent Literature 2. These literatures describe a method where a used heteropoly acid-based catalyst for manufacturing methacrylic acid is dissolved and a recovered solid portion is used as a raw material of a catalyst for manufacturing methacrylic acid and a method where at least one kind selected from a cesium salt, a potassium salt, a thallium salt and a rubidium salt is added to a recovered liquid portion to form a precipitate, which is then used as a raw material of a catalyst for manufacturing methacrylic acid, and it is described that the recovered solid content is regenerated to have performance equivalent to an unused catalyst by the methods described in these descriptions.
In addition, Patent Literature 3 shows a method for manufacturing a catalyst where a catalyst containing molybdenum used in reaction is dispersed in water and an alkali metal compound and/or ammonia water is added for adjusting the pH to 6.5 or less to form a precipitate, which is then used as a raw material of a catalyst for manufacturing methacrylic acid.
Patent Literature 4 describes that a heteropoly acid-based catalyst having reduced activity after used in reaction is dissolved and/or decomposed in an aqueous medium, and treated with an inorganic ion exchanger to prepare a catalyst having performance equivalent to an unused catalyst, which also has a catalyst lifetime closer to an unused catalyst.

### Related Technical Literature

### Patent Literature

[Patent Literature 1] Japanese Patent Laid-Open No. 2008-710 A1
[Patent Literature 2] Japanese Patent Laid-Open No. 2008-709 A1
[Patent Literature 3] Japanese Patent No. 3887511 A1
[Patent Literature 4] Japanese Patent No. 3298978 A1

### Disclosure of the Invention

### Problems to Be Solved by the Invention

Any of the recovered catalysts described in the above prior arts is intended to be a used catalyst of a gas-phase oxidation catalyst and there is no description about a recovered catalyst from a catalyst manufacturing process. In a catalyst manufacturing process, mainly in a drying process with a spray dryer, a molding process, a calcining process and the like, some catalyst is lost out of the processes due to catalyst scattering and adhering to a container, resulting in catalyst loss to some extent. The addition and the like are carried out by calculating the loss from the beginning and catalyst lost in those processes has been conventionally discarded. In addition, a catalyst failed due to problems in quality and the like, a product in process (including a semi-finished product and the like) which is surplus as a fraction associated with the addition, and the like have been conventionally discarded in many cases. However, there is an increasing requirement for effective utilization of a waste catalyst generated in these manufacturing processes, from a viewpoint of environmental problems and effective utilization of resources. So the present inventors recovered such an unused waste catalyst, for the purpose of effective utilization; again dissolved said unused recovered catalyst in water; separated out an insoluble impure portion which had come to be mixed in a scattered catalyst- recovering process and insoluble carriers and the like contained in the catalyst, to give an aqueous solution containing said recovered catalyst component; dried this with a spray dryer to give catalyst granules; and regenerated a catalyst for methacrolein oxidation through molding and calcining processes, resulting in that the desired performance was not achieved even though it was an unused catalyst. With that, the present inventors have found that there is still a problem in using it as mixed with a new catalyst.

That is, an unused recovered catalyst from a manufacturing process and/or a used catalyst recovered for regeneration (hereinafter, also simply referred to as recovered catalyst, including the both) are usually filled in a reactor for manufacturing methacrylic acid which is equipped with several tens of thousands of reaction tubes at the same time as an unused catalyst is filled. Then, when there is difference in performance such as activity between said regenerated catalyst and the unused catalyst, the both are filled in the reaction tubes as they are to generate variation in activity between several tens of thousands of reaction tubes due to difference in reaction between the both, leading also variation in catalyst lifetime and occurrence of inconvenience such as reduction in the total yield. Because of that, there is necessity to homogenously mix the both in order to use them together, which is an operation unnecessary when using only an unused catalyst. It is also a very difficult operation to homogenously mix a great deal of catalyst at an industrial scale, so said regenerated catalyst is required to have performance equivalent to an an unused catalyst. Because of that, any reuse of a used catalyst described in the above prior art is usually carried out for the purpose of regenerating so that it has performance as equivalent to a new catalyst as possible. And, in regeneration from a used catalyst in these prior arts, for example, Patent Literatures 1 to 3 and the like reuse a heteropoly acid catalyst component once dissolved, after recovering as a precipitate (solid matter). However, in order to carry out recovery of a solid matter containing water at an industrial scale, there are problems such as necessity of a device for centrifugal separation and decantation for a long period of time.
In addition, the method in Patent Literature 4 gives highly active regeneration by treatment with an ion exchanger, which is however not necessarily satisfying the purpose of giving the same performance as an unused catalyst.

### Means of Solving the Problems

The present inventors have intensively studied under such a situation and found that a heteropoly acid-based catalyst without the above-described problem can be manufactured by dispersing and/or dissolving a substance intended for regeneration (a catalyst discarded due to scattering and the like in the above manufacturing process, a catalyst failed and discarded due to problems in quality and the like, an unused recovered catalyst such as a product in process which is surplus as a fraction associated with the addition or filling, or a recovered used catalyst) in an aqueous solvent to obtain a solution part, which is then treated as it is in solution in a certain process to obtain a solution without separation of the catalyst component as a solid content, followed by manufacturing a catalyst therefrom; and the present invention has been thus completed. That is, is found that said method can be likewise applied to manufacture a catalyst not only in the case of using an unused recovered catalyst from the above manufacturing process but also in the case of using a used catalyst as a raw material.

That is, the present invention relates to:
(1) A method for manufacturing a catalyst, which is characterized by comprising the following processes for manufacturing a regenerated heteropoly acid-based catalyst using, as a raw material, recovered heteropoly acid-based catalyst containing molybdenum, phosphorus, vanadium or copper as an essential component:
   Process a): a solution A is prepared by mixing a heteropoly acid-based catalyst with an aqueous solvent and removing a component insoluble in the solvent;
   Process b): the molar quantity of at least one component of molybdenum, phosphorus, vanadium or copper contained in the solution A is measured;
   Process c): an aqueous hydrogen peroxide solution is added to the solution A or a solution obtained in the below-described Process d to oxidize the heteropoly acid;
   Process d): the difference between the above molar quantity obtained in Process b) and the mol theoretical value of an element required for preparation of an target regenerated catalyst is determined and the shortage amount of a catalyst component is added to the solution A or the solution obtained in Process c to prepare a solution containing the additional raw material component;
   Process e): the solution obtained through Process a to Process d is dried to prepare catalyst granules D; and
   Process f): the catalyst granules D are molded and subsequently calcined to prepare a molded catalyst E.
(2) The manufacturing method according to the above-described (1), wherein the heteropoly acid-based catalyst further contains one kind or more selected from the below-described X and/or one kind or more selected from Y and wherein the content of at least one component of molybdenum, phosphorus, vanadium, copper, X or Y is measured in Process b):
   X: alkali metal, alkali earth metal or ammonia;
   Y: silver, zirconium, arsenic, boron, germanium, tin, lead, chrome, bismuth, cobalt, nickel, cerium, tungsten, iron, aluminum, magnesium, antimony, niobium, manganese or titanium.
(3) The method for manufacturing a catalyst according to the above-described (1) or (2), wherein the above heteropoly acid-based catalyst is a catalyst represented by the below-described general formula:

   MoₐP_{b}V_{c}Cu_{d}XₑY_{f}O_{g}

   (wherein, Mo, P, V and Cu respectively represent molybdenum, phosphorus, vanadium and copper; X represents at least one kind selected from the group consisting of an alkali metal, an alkali earth metal and ammonia and Y represents at least one kind selected the group consisting of silver, zirconium, arsenic, boron, germanium, tin, lead, chrome, bismuth, cobalt, nickel, cerium, tungsten, iron, aluminum, magnesium, antimony, niobium, manganese and titanium, respectively; each symbol of a to g is an atomic ratio of the element, where b is 0.1 to 6, c is 0.1 to 6, d is 0.1 to 4.0, e is 0 to 4, is 0 to 5 when a = 10, and g is a numerical value determined depending on the oxidation state of each element).
(4) The method for manufacturing a catalyst according to any one of the above-described (1) to (3), which is characterized in that the heteropoly acid-based catalyst is a catalyst for manufacturing methacrylic acid by gas-phase partial oxidation reaction of methacrolein.
(5) The method for manufacturing a catalyst according to any one of the above-described (1) to (4), which is characterized in that the heteropoly acid-based catalyst is a recovered product of a waste catalyst generated in a process for manufacturing a gas-phase oxidation catalyst for manufacturing methacrylic acid from methacrolein or a recovered product of a product in in process of said gas-phase oxidation catalyst.
(6) The method for manufacturing a catalyst according to any one of the above-described (2) to (5), which is characterized in that X is cesium and/or ammonia.
(7) The method for manufacturing a catalyst according to any one of the above-described (2) to (6), wherein Y is antimony and/or arsenic.
(8) The method for manufacturing a catalyst according to any one of the above-described (2) to (5), wherein the catalyst is a catalyst not containing X.
(9) The method for manufacturing a catalyst according to any one of the above-described (3) to (5) or (7), wherein e is 0, and Y is at least one element selected from the group consisting of arsenic, antimony and cerium.
(10) The method for manufacturing a catalyst according to any one of the above-described (1) to (9), which is characterized in that the molded catalyst E is a molded catalyst where an inactive carrier is coated with the catalyst granules D using a liquid binder.

### Effect of the Invention

According to the present invention, it is possible that a recovered heteropoly acid-based substance (specifically, a recovered heteropoly acid-based catalyst) is, with good reproducibility, regenerated into a catalyst having almost the same performance as an unused catalyst. Therefore, it is possible to reuse a waste catalyst conventionally generated in a manufacturing process, for example, a catalyst lost due to scattering or the like and discarded; a catalyst failed due to problems in quality and the like and discarded and a catalyst being a fraction associated with filling or the like and discarded (which is technically not a waste catalyst generated in a manufacturing process but included here because it is in common in terms of unused waste catalyst, in the present invention); or a recovered product (unused recovered catalyst) of an unused catalyst such as a product in process (also including a semi-finished product) which has been a fraction associated with the addition or the like and it is possible to effectively reuse a used catalyst, leading to improvement in basic unit of catalyst production.

### Mode for Carrying Out the Invention

Hereinafter, the present invention will be specifically explained.
The recovered heteropoly acid-based catalyst (substance intended for regeneration) (hereinafter, also referred to as substance M) containing molybdenum, phosphorus, vanadium or copper and preferably these 4 elements as an essential component, which is used as a raw material in the present invention, can include a recovered heteropoly acid-based catalyst containing molybdenum, phosphorus, vanadium and an essential component, which is used in gas-phase oxidation reaction. Said substance M can specifically include a catalyst lost from a process, specifically a catalyst manufacturing process, due to scattering, adhering to a container and the like and conventionally discarded (a product in process including a semi-finished product, a final catalyst and the like); an unused recovered catalyst such as products in process (also including semi-finished a final catalyst and/or the like which have discarded due to problems in quality, addition, and the like (specifically, a waste catalyst generated in a process for manufacturing a gas-phase oxidation catalyst or a recovered product of a product in process); a catalyst used in oxidation reaction and deteriorated (used catalyst); and the like.
In this regard, the term "product in process" mentioned in the present description means a catalyst containing a catalyst component (preferably, containing all the active components) of an target catalyst but not being a complete target catalyst, and can include, for example, a slurry containing a catalyst components, a dried form thereof, a coated catalyst which has not been calcined, a calcined catalyst which has finished with a first calcination (semi-finished product); and the like.
The substance M may comprise only a catalyst active component but usually comprises a catalyst active component, an inactive component such as a carrier other than the catalyst active component and the like, an impurity which has come be mixed in recovering, and the like.
The reaction can include gas-phase oxidation reaction for oxidation of methacrolein to obtain methacrylic acid, and as the substance M used as a raw material in the invention, a catalyst and a used catalyst which have recovered from a process for manufacturing a heteropoly acid catalyst used in said reaction are particularly preferable.

The heteropoly acid catalyst intended for regeneration may further contain an active component other than the above-described essential components, if necessary. The other active component and its use ratio is appropriately determined depending on the use conditions of the catalyst so that the catalyst exhibiting optimum performance is obtained. Said other active component can include, for example, the below-described X and/or Y:
X: at least one kind selected from the group consisting of an alkali metal, an alkali earth metal and ammonia; and
Y: at least one kind selected from the group consisting of silver, zirconium, arsenic, boron, germanium, tin, lead, chrome, bismuth, cobalt, nickel, cerium, tungsten, iron, aluminum, magnesium, antimony, niobium, manganese and titanium.
At the above-described X component, cesium and/or ammonia are preferable. As the above-described Y component, arsenic, antimony and/or cerium are preferable. A catalyst containing these preferable X and/or Y components is one of the preferable catalysts. A catalyst containing no X component is one of the preferable catalysts. A more preferable catalyst is a catalyst which contains no X no X and Y components or which contains no X component and contains at least one kind selected from the group consisting of arsenic, antimony and cerium as Y component (preferably, Y component is either one or the both of arsenic or antimony).

A specific catalyst of the above heteropoly acid catalyst can include a catalyst represented by the below-described general formula:

MoₐP_{b}V_{c}Cu_{d}XₑY_{f}O_{g}

(wherein, P, V and Cu respectively represents molybdenum, phosphorus, vanadium and copper; X represents at least one element (or molecules) selected from an alkali metal, an alkali earth metal and ammonia and Y represents at least one element selected from the group consisting of silver, zirconium, arsenic, boron, germanium, tin, lead, chrome, bismuth, cobalt, nickel, cerium, tungsten, iron, aluminum, magnesium, antimony, niobium, manganese or titanium, respectively; and subscripts on the right of element symbols are each an atomic ratio of each element, where b is 0.1 or more and 6 or less and preferably 0.3 or more and 4.0 or less, c is usually 0.1 or more and 6 or less and preferably 0.3 or more and 3.0 or less, d is usually 0.1 or more and 4.0 or less and preferably 0.2 or more and 1.0 or less, e is usually 0 or more and 4 or less, f is usually 0 or more and 5 or less when a = 10, and g is a numerical value determined depending on the oxidation state of each element).
In the above-described general formula, preferable X component is cesium and/or ammonia. Preferable Y component is at least one kind selected from the group consisting of arsenic, antimony and cerium. One of the preferable catalysts is a catalyst containing a preferable component described above as X and/or Y, as described above. In addition, one of the more preferable catalysts is a catalyst not containing X in the above-described general formula and further preferably is a catalyst where Y is a component listed above as preferable. Most preferable is a catalyst where in the above-described general formula, no X component is contained and Y component is at least one kind selected from the group consisting of arsenic, antimony and cerium (preferably, Y component is either one or the both of arsenic or antimony).

Hereinafter, preferable embodiments will be described with respect to each process.

### Process a)

First, in Process a), a substance M as a substance intended for regeneration is mixed with an aqueous solvent and a component insoluble to the solvent is removed to prepare a solution A containing an active component element of the above-described catalyst.
The catalyst component contained in the M is generally highly soluble to water, but when an insoluble carrier and an impurity such as insoluble foreign matter which has come to be mixed in recovering are contained, they are not dissolved but dispersed. In the present invention, the substance M is preferably a heteropoly acid catalyst where a water-insoluble component is not included in the catalyst active component.
As the aqueous solvent used in the present invention, ion-exchanged water and/or distilled water containing no organic solvent is usually suitable. However, in some cases, ethanol or the like can be appropriately added to this for for use. By adding ethanol, solubility may be improved in some cases. Therefore, in some cases, an aqueous solvent such as a water-ethanol mixed solvent is also preferable.
The amount of a solvent to be used is, preferably, approximately 0.5 time to 2 times the weight of the substance M. When the amount of the solvent is too small, said catalyst component may not sufficiently dissolve, and when it is too large, effects appropriate to it are hardly obtained.

The substance M and the aqueous solvent may be mixed by any method as long as the both can be homogenously mixed. The mixing can be carried out by gradually adding the substance M usually while stirring the aqueous solvent. It can be usually carried out at ordinary temperature.
The mixing time of the substance M with the aqueous solvent is not particularly limited as long as a recovered catalyst component in the substance M can be dissolved. The heteropoly acid catalyst component in the substance M is easily dissolved in water, so approximately 1 minute to 30 minutes is usually preferable. In the meantime, it is preferred to stir to the extent that the content in a dissolver becomes homogenous. After stirring, the solution part and the insoluble component are separated. They may be separated immediately after stirring or may be separated after leaving to stand for approximately 1 minute to 30 minutes. This separation of the solution part and the insoluble portion can be carried out by a common method for usual solid-liquid separation. For example, most generally, the insoluble portion can be separated and removed by filtration. When the insoluble portion is left to stand for precipitation, the insoluble portion may be removed by a combination of pumping and filtering the solution part as a supernatant. The insoluble portion is allowed to be discarded as it is, but in order to improve the recovery yield of a catalyst component remaining in an insoluble portion after filtration, an operation of washing the insoluble portion after filtration with an aqueous solvent (preferably, water) may be repeated approximately one to five times and preferably approximately one to three times or an operation of again mixing said insoluble portion with an aqueous solvent (preferably, water) followed by separation may be repeated approximately one to five times (preferably, approximately one to three times). Usually, by repeating, approximately one to three times, washing of the above-described insoluble portion with an aqueous solvent or by mixing with an aqueous solvent followed by separation, the recovery yield of a catalyst component typified by molybdenum contained in the insoluble portion is improved. Therefore, it is preferred to carry out the above-described operation a plurality of times. A solution portion obtained by these operations can be combined with the solution part obtained in the first operation to give a solution A containing the catalyst component.
Too low solute concentration in the solution A results in reduction in catalyst recovery efficiency, so the solute concentration in the solution A is preferably approximately 10 to 40% by weight and more preferably approximately 20 to 30% by weight based on the total amount of the solution A.

### Process b)

Process b) is a process for measuring the molar quantity of at least one component of molybdenum, phosphorus, vanadium or copper contained in the solution A.
When the substance M is a catalyst comprising a water-soluble heteropoly acid containing no water-insoluble salt such as a cesium salt, particularly a catalyst recovered from the manufacturing process (also referred to as recovered catalyst from manufacturing process), the catalyst should have the same composition ratio as an unused catalyst because it has not been used in reaction. However, under the present inventors' study, the regenerated catalyst manufactured after dissolving said process recovered catalyst did not exhibit the same performance as an unused catalyst. By pursuing the cause, it was found that one of the causes was that the composition ratio of molybdenum, phosphorus, vanadium or copper and the optional component contained in the solution (A) had been different from the composition ratio of the target catalyst regenerated. For that reason, in order that the performance of a regenerated catalyst is equivalent to that of an unused catalyst, it is required that the concentration of a catalyst component contained in a solution A is subjected to quantitative analysis and the composition ratio of active component elements in a regenerated catalyst is in line with that of an target catalyst.
The measurement of the concentration of catalyst components in a (solution A can be carried out by a known method. For example, a solution for measurement is taken from a solution A and the concentration of the catalyst component contained in the solution for said measurement can be quantitatively analyzed by ICP spectrometry, atomic absorption spectrometry, fluorescence X-ray analysis or the like. These methods are preferable in terms that simple and accurate measurement can be carried out.
When a deficient component is found in advance, quantitative analysis of the catalyst component may be conducted only on the component, but usually, it is preferred that quantitative analysis is carried out on all active component elements in an target catalyst because excess and deficiency in the content of an active component element in a recovered catalyst cannot be predicted.

### Process c)

Process c) is a process for adding an aqueous hydrogen peroxide solution to the solution A to oxidize a recovered catalyst component (heteropoly acid) (where this process is, for convenience, also referred to as hydrogen peroxide addition process or process for obtaining a solution (B)).
In spite that a recovered catalyst from a manufacturing process is unused, a solution A obtained by dissolving said catalyst exhibits a dark green to blue color which is a reduced color of heteropoly acid. Under the present inventors' study, it is difficult to obtain a regenerated catalyst having performance equivalent to an unused catalyst by using this solution as it is. However, by adding an aqueous hydrogen peroxide solution to said solution (A) to oxidize a recovered heteropoly acid, the active component performance in a recovered catalyst can be returned to the active component performance in a target unused catalyst.
The concentration of an aqueous hydrogen peroxide solution to be used is usually 5 to 30% by weight. It is not necessarily appropriate to suggest the amount of a hydrogen peroxide to be used because it varies depending on the composition of the heteropoly acid and the history of the substance M, but it is approximately 5 to 20% by weight to the substance M.
There is a tendency that a higher temperature of a solution A leads to reduction in use amount. Oxidation reaction with an hydrogen peroxide solution may be carried out at ordinary temperature, but it is preferred to beforehand raise the temperature of a solution A in the range of approximately 40 to 100°C, preferably approximately 40 to 95°C and more preferably approximately 60 to 95°C and then to gradually add an aqueous hydrogen peroxide solution so as to maintain the temperature. Oxidation reaction with a hydrogen peroxide is associated with heat generation, so it should be carefully conducted so that the temperature of a (solution A is not raised too high.
A solution obtained in the above-described oxidation with an aqueous hydrogen peroxide solution is defined as an oxidized solution (solution B).
With regard to the above-described Process b and Process c, Process c may be first conducted followed by Process b.

### Process d)

Process d is a process for allowing the composition ratio of catalyst active component elements contained in a solution A to correspond to the composition ratio (theoretical value) of active component elements in an target catalyst, using the quantitative analysis results in Process b (where this process is also referred to as component-adjusting process).
From comparison of the composition ratio of catalyst active component elements in said quantitative analysis results with the composition ratio of active component elements in an target catalyst, an active component element to be added to a solution A (or solution B) and an addition amount thereof are calculated.
A component to be added to a solution A (or solution B) (additional component) in an additional amount calculated from difference between quantitative analysis results in Process b and the theoretical value of an target catalyst composition is added as an additional raw material. As the additional raw material, a compound containing an additional component element can be appropriately selected. It is preferred to use the same material compound as that used for manufacturing an target catalyst.
In this regard, the above theoretical value corresponds to the addition molar ratio of the raw material elements upon manufacturing an target catalyst. In addition, the concentration of a solute (the total concentration of a catalyst component) contained in a solution obtained after putting and dissolving an additional raw material is preferably 5 to 15% by weight and more preferably approximately 10% by weight (for example, approximately 8 to 12% by weight) based on the total amount of said solution. According to necessity, It is preferred to adjust the solute concentration in said solution to the above-described range by adding deionized water. With regard to the above-described Process c (hydrogen peroxide addition process) and Process d (component-adjusting process), Process d may be first conducted followed by Process c.
As is clear from the above, the order of Process b to Process d may be not necessarily the same as described above. For example, the order of carrying out Process b and Process c can be exchanged and the order of carrying out Process c and Process d can be exchanged.

### Process e)

By drying the solution (also referred to as solution C) obtained through the above-mentioned Process a) to Process d) by a known method, catalyst granules D can be obtained. Drying means is not particularly limited but it is preferable to dry using, for example, a spray dryer.

### Process f)

This is a process for molding the catalyst granules D to obtain a molded catalyst E.
The shape of the molded catalyst E can be appropriately selected from shapes such as cylindrical, tablet, spherical and ring shapes, for example. The molding method is not particularly limited and a molding method of an oxidation catalyst which is used for manufacturing (meth)acrylic acid, for example, a method such as extrusion granulation, tableting and and coating methods can be employed. In the case of a coating method, a catalyst active component can be supported on an approximately 2 to 4 mm spherical carrier, particularly an inactive carrier such as silica and alumina, according to necessity, together with a binder of water or/and an organic binder (for example, ethanol and the like) to give a a supported catalyst having a particle size of 3 to 6 mm. In the present invention, said supported catalyst is preferable in terms of reaction performance, heat-removing efficiency and the like.
In this regard, when said process recovered catalyst is a supported catalyst and an organic binder is used in a supporting process, a substance M contains a carrier or an organic binder in some cases, where there is no particular harm in the present invention. For example, a water-soluble organic binder, for example, an aqueous ethanol solution may be removed by heating a solution A before adding a hydrogen peroxide in Process c. In addition, an insoluble binder such as a carrier is recovered as an insoluble portion in Process a.

The molded catalyst E regenerated according to the present invention can be used alone or together with an unused target catalyst. Preferably by a known method, it is used for gas-phase partial oxidation reaction of methacrolein. For example, it is preferred that said molded catalyst E is filled alone or together with an unused target catalyst in a shell-and-tube reactor so that the layer height is 2 m to 5 m, a gas containing 2% by volume to 6% by volume of methacrolein and coexisting with oxygen 2.0 molar times based on said methacrolein and with water vapor 3.0 molar times or more based on said methacrolein is contacted with the catalyst at a space velocity of 600 h⁻¹ to 1800 h⁻¹, and gas-phase partial oxidation reaction of methacrolein is carried out at a reaction temperature of 260°C to 360°C under an atmosphere to an atmospheric pressure of approximately 100 kPaG. The methacrolein to be used is not necessarily a pure product and may contain carbon monoxide, carbon dioxide, aldehyde, carboxylic acid and an aromatic compound as an organic impurity.
It is not necessarily appropriate to suggest because the use period of the catalyst varies depending on the above reaction conditions, but it is usually 1 year to 4 years.

As the above-mentioned, the substance M may be a used catalyst (deteriorated catalyst) used in a process for manufacturing methacrylic acid by oxidation reaction, preferably gas-phase partial oxidation reaction of methacrolein or may be an unused recovered catalyst such as a waste catalyst generated in a process for manufacturing a gas-phase oxidation catalyst for manufacturing methacrylic acid from methacrolein or a recovered product of a product in process. The waste catalyst generated in a manufacturing process can include an unused catalyst (waste catalyst) which is conventionally treated as a waste as is mentioned above. For example, it can include a catalyst lost from a catalyst manufacturing process (for example, a catalyst lost due to scattering, adhering to a container, or the like, for example, a catalyst such as a product in process adhering to a container or the like, a product in process scattering during spray drying, and a semi-finished product adhering to a container or the like before calcination or having finished with the first calcination) or a catalyst which has been surplus as a fraction associated with filling or the like and discarded. In addition, the product in process of gas-phase oxidation catalyst can include a product in process which has been surplus as a fraction associated with the addition or the like, and the like.
In this regard, in the present description, the above "theoretical value" corresponds to the addition molar ratio of a raw material element for manufacturing a target catalyst.

Hereinafter, preferable aspects of the present invention (the invention described in (1) of the above Means of Solving the Problems) will be summarized as follows:
(I) An aspect in which a heteropoly acid-based catalyst recovered for regeneration is a recovered catalyst from manufacturing process or a used catalyst;
(II) The aspect according to the above-described (I), wherein the above-described catalyst is a catalyst for manufacturing methacrylic acid by gas-phase partial oxidation reaction of methacrolein;
(III) The aspect according to the above-described (I) or (II), wherein the above-described catalyst is a catalyst of the above general formula (1) where e is 0 and Y is at least one element selected from the group consisting of arsenic, antimony and cerium;
(IV) The aspect according to the above-described (III), wherein Y is arsenic or antimony in the above general formula (1);
(V) The aspect according to any one of the above-described (I) to (IV), wherein the above-described catalyst is a heteropoly acid catalyst represented by the above general formula (1) where b is 0.3 to 4.0, c is 0.3 to 3.0, d is 0.2 to 1.0, e is 0 and f represents the total amount of Y and is 0 to 3, when a = 10;
(VI) The aspect according to any one of the above-described (I) to (V), wherein the aqueous solvent in the above Process a is water;
(VII) The aspect according to any one of the above-described (I) to (VI), wherein Process b or Process c and Process c or Process d are carried out in an arbitrarily order except that the above Process d is carried out after Process b;
(VIII) The aspect according to any one of the above-described (I) to (VII), wherein the above Processes a to e are carried out in the order of a, b, c, d and e; and
(IX) The aspect according to any one of the above-described (I) to (VIII), wherein the molded catalyst E is a supported catalyst.

### Examples

Hereinafter, the present invention will be more specifically explained with reference to Examples. In this regard, the present invention is not limited to the Examples unless the purposes depart from the purpose of the present invention. In the following Examples, the conversion ratio and yield are defined as follows:
· methacrolein conversion ratio = {(molar number of methacrolein supplied - molar number of methacrolein unreacted)/ molar number of methacrolein supplied} × 100;
· methacrylic acid yield = {( molar number of methacrylic acid generated - molar number of methacrylic acid supplied)/ molar number of methacrolein supplied} × 100; and
· methacrylic acid selectivity = {(molar number of methacrylic acid generated - molar number of methacrylic acid supplied)/(molar number of methacrolein supplied - molar number of methacrolein unreacted)} x 100.

### Example 1

To 10000 ml of distilled water of room temperature, 1000 g of molybdenum trioxide, 96.09 g of a 85 wt.% aqueous phosphoric acid solution, 37.91 g of vanadium pentaoxide, 65.73 g of a 60 wt.% aqueous arsenic acid solution and 22.1 g of cupric oxide were added, the temperature was raised to 95°C while stirring, and the mixture were dissolved at 95°C over 10 hours under reflux by heating to obtain a red-brown solution. This was dried with a spray dryer to obtain catalyst granules.
With 500 parts by weight of the obtained catalyst granules, 70 parts by weight of strength enhancer were mixed. This was supported on 430 parts by weight of a silica/alumina inactive carrier (having a particle size of 3.5 mm) with an 80 wt.% aqueous ethanol solution as a binder.
The thus-obtained molded substance was calcined at 300°C for 5 hours to obtain a heteropoly acid catalyst 1 (which is referred to as hereinafter catalyst 1). The average particle size of the obtained catalyst was 4.3 mm. The obtained heteropoly acid catalyst had a composition ratio except for oxygen consisting of: 0.6 of vanadium, 1.2 of phosphorus, 0.4 of arsenic and 0.4 of copper, based on 10 of molybdenum.

The above-described catalyst manufacturing was successively carried out, and meanwhile a catalyst (substance M) lost from the drying process with a spray dryer, the molding process and the calcining process mainly due to scattering, adhering to a container, and the like was recovered. To 1000 g of deionized water, 1000 g of the substance M was added, and the mixture was stirred for 3 minutes and then filtered by suction using a filter paper. While the filtration residue was allowed to remain in the nutsche, 1000 g of deionized water were added and again the operation of filtration by suction was carried out three times in total. The obtained filtrates were all combined to obtain a filtrate (solution A). The finally-remaining solid content was about 200 g and had silica and alumina as a main component as determined in qualitative analysis by fluorescence X-ray analysis.

The weight of the filtrate (solution A) recovered in the above process was measured, 100 g thereof was sampled, and the rest was transferred into a jacket-type furnace and heated with stirring at 90°C for 15 minutes.
The sampled filtrate (solution A) was dried in an evaporator and the obtained powder was subjected to quantitative analysis by fluorescence X-ray analysis, resulting in that the component and weights thereof to be added for returning to the theoretical value of the target catalyst before using were: 12.0 g of molybdenum trioxide, 2.5 g of vanadium pentaoxide and 0.7 g of cupric oxide.
To the rest solution A after sampling, 5000 g of deionized water were added, and further 300 ml of a 30 wt.% aqueous hydrogen peroxide solution were gradually added at 90°C while heating the mixture with stirring, resulting in that the dark green solution A turned to an orange yellow transparent solution B. To the solution B, 12.0 g of molybdenum trioxide, 2.5 g of vanadium pentaoxide and 0.7 g of cupric oxide were added and the mixture was heated with stirring at 90°C for 30 minutes to obtain a solution C. This solution C was dried with a spray dryer to obtain catalyst granules.
Then, by the same method as that of manufacturing the above-described catalyst 1, a regenerated supported catalyst (catalyst E1) was manufactured.

### (Partial oxidation reaction of methacrolein)

Each 10.3 ml of the catalyst 1 and the catalyst E1 were filled in a stainless reaction tube having an internal diameter of 18.4 mm. Through said reaction tube, a material gas (composition (molar ratio); methacrolein: oxygen: water vapor: nitrogen = 1:2:4:18.6) was passed at a space velocity (SV) of 1200 h⁻¹ and oxidation reaction of methacrolein was carried out as described below.
The oxidation reaction was first continued at a reaction bath temperature of 310°C for 3 hours, and subsequently the reaction bath temperature was raised to 350°C and the reaction was continued for 15 hours (hereinafter, this treatment is referred to high temperature reaction treatment). Subsequently, the reaction bath temperature was lowered to 310°C and the reaction performance was measured.
In this regard, the high temperature reaction treatment is an acceleration test to see deterioration and the like of catalyst activity in a severe test.
The oxidation reaction results of the catalyst 1 and the catalyst E1 are shown in Table 1.

### Example 2

To 10000 ml of distilled water of room temperature, 1000 g of molybdenum trioxide, 88.1 g of a 85 wt.% aqueous phosphoric acid solution, 37.9 g of vanadium pentaoxide, 82.2 g of a 60 wt.% aqueous arsenic acid solution, 55.5 g of cupric acetate and 10.2 g of antimony trioxide were added, the temperature was raised to 95°C while stirring, and the mixture was dissolved under reflux by heating at 95°C over 10 hours to obtain a red-brown solution. This was dried with a spray dryer to obtain catalyst granules.
With 500 parts by weight of the obtained catalyst granules, 70 parts by weight of a strength enhancer were mixed. This was supported on 430 parts by weight of a silica/alumina inactive carrier (having a particle size of 3.5 mm) with a 80 wt.% aqueous ethanol solution as a binder.
The thus-obtained molded substance was calcined at 300°C for 5 hours to obtain a heteropoly acid catalyst (hereinafter, referred to as catalyst 2). The average particle size of the obtained catalyst was 4.3 mm. The obtained catalyst 2 has a composition ratio except for oxygen consisting of: 0.6 of vanadium, 1.1 of phosphorus, 0.5 of arsenic, 0.4 of copper and 0.1 of antimony, based on 10 of molybdenum.

The above-described catalyst manufacturing was successively carried out, and meanwhile a catalyst (substance M) lost from the drying process with a spray dryer, the molding process and the calcining process mainly due to scattering, adhering to a container, and the like was recovered. To 1000 g of deionized water, 1000 g of the substance M were added, and the mixture was stirred for 3 minutes and then filtered by suction using a filter paper. While the filtration residue was allowed to remain in the nutsche, 1000 g of deionized water were added and again the operation of filtration by suction was carried out twice in total. The obtained filtrates were all combined to obtain a filtrate (solution A). The finally-remaining solid content was about 200 g and had silica and alumina as a main component as determined in qualitative analysis by fluorescence X-ray analysis.

The weight of the filtrate (solution A) recovered above was measured, 100 g thereof was sampled, and the rest was transferred into a jacket-type furnace and heated with stirring at 90°C for 15 minutes.
The sampled filtrate (solution A) was dried in an evaporator and the obtained powder was subjected to quantitative analysis by fluorescence X-ray analysis, resulting in that the components and weights thereof to be added for returning to the theoretical value of the target catalyst before using were: 27.0 g of molybdenum trioxide, 2.0 g of vanadium pentaoxide and 2.5 g of cupric acetate.
The solution A had a smell of ethanol, so 6000 g of deionized water were also added to the solution A and then the mixture was further heated with stirring at 90°C for 2 hours. After that, 300 ml of a 30 wt.% aqueous hydrogen peroxide solution were gradually added while continuously stirring, resulting in that the dark green solution A turned to an orange yellow transparent solution B. To the solution B, 27.0 g of molybdenum trioxide, 2.0 g of vanadium pentaoxide and 2.5 g of cupric acetate were added, and the mixture was heated with stirring at 90°C for 90 minutes to obtain a solution C. This solution C was dried with a spray dryer to obtain catalyst granules.
Then, by the same method as that of manufacturing the above-described catalyst 2, a regenerated supported catalyst (catalyst E2) was manufactured. In this regard, it is inferred that the cause of the smell of ethanol from the solution A is that most of the substance M used were from a process for molding a catalyst.
In the same manner as in Example 1, partial oxidation reaction of methacrolein was carried out using the catalyst 2 and the catalyst E2. The results are shown in Table 1.

### Example 3

To 10000 ml of distilled water of room temperature, 1000 g of molybdenum trioxide, 112.1 g of a 85 wt.% aqueous phosphoric acid solution, 75.8 g of vanadium pentaoxide and 22.11 g of cupric oxide were added, the temperature was raised to 95°C while stirring, and the mixture was dissolved under reflux by heating at 95°C over 10 hours to obtain red-brown solution. This was dried with a spray dryer to obtain catalyst granules.
With 500 parts by weight of the obtained catalyst granules, 70 parts by weight of a strength enhancer were mixed. This was supported on 430 parts by weight of a silica/alumina inactive carrier (having a particle size of 3.5 with a 80 wt.% aqueous ethanol solution as a binder.
The thus-obtained molded substance was calcined at 300°C for 5 hours to obtain a heteropoly acid catalyst 2. The average particle size of the obtained catalyst was 4.3 mm. The obtained heteropoly acid catalyst had a composition ratio except for oxygen consisting of: 1.2 of vanadium, 1.4 of phosphorus and 0.4 of copper, based on 10 of molybdenum.

The above-described catalyst manufacturing was successively carried out, and meanwhile a catalyst (substance M) lost from the drying process with a spray dryer, the molding process and the calcining process mainly due to scattering, adhering to a container, and the like was recovered. To 1000 g of deionized water, 1000 g of the substance M was added, and the mixture was stirred for 3 minutes and then filtered by suction using a filter paper. While the filtration residue was allowed to remain in the nutsche, 1000 g of deionized water were added and again the operation of filtration by suction was carried out three times in total. The obtained filtrates were all combined to obtain a filtrate (solution A). The finally-remaining solid content was about 200 g and had silica and alumina as a main component as determined in qualitative analysis by fluorescence X-ray analysis.

The weight of the filtrate (solution A) recovered in the above process was measured, 100 g thereof was sampled, and the rest was transferred into a jacket-type furnace and heated with stirring at 90°C for 15 minutes.
The sampled filtrate (solution A) was dried in an evaporator and the obtained powder was subjected to quantitative analysis by fluorescence X-ray analysis, resulting in that the component and weights thereof to be added for returning to the theoretical value of the target catalyst before using were: 27.0 g of molybdenum trioxide, 2.0 g of vanadium pentaoxide and 2.5 g of cupric acetate.
The solution A had a smell of ethanol, so 5000 g of deionized water were added to the solution A and then the mixture was further heated with stirring at 90°C for 2 hours. After that, 300 ml of a 30 wt.% aqueous hydrogen peroxide solution were gradually added while continuously stirring, resulting in that the dark green (solution A turned to an orange yellow transparent solution B. To the solution B, 27.0 g of molybdenum trioxide, 2.0 g of vanadium pentaoxide and 2.5 g of cupric acetate were added, and the mixture was heated with stirring at 90°C for 90 minutes to obtain a solution C. This solution C was dried with a spray dryer to obtain catalyst granules.
From then on, by the same method as that of manufacturing the above-described catalyst 3, a regenerated supported catalyst (catalyst E3) was manufactured.
In the same manner as in Example 1, partial oxidation reaction of methacrolein was carried out using the catalyst 3 and the catalyst E3. The results are shown in Table 1.

### Example 4

To 20000 ml of distilled water of room temperature, 2000 g of molybdenum trioxide, 192.18 g of a 85 wt.% aqueous phosphoric acid solution, 75.82 g of vanadium pentaoxide, 131.46 g of a 60 wt.% aqueous arsenic acid solution and 44.2 g of cupric oxide were added, the temperature was raised to 95°C while stirring, and the mixture was dissolved under reflux by heating at 95°C over 10 hours to obtain a red-brown solution. This was dried with a spray dryer to obtain catalyst granules.
With 1000 parts by weight of the obtained catalyst granules, 140 parts by weight of a strength enhancer were mixed. This was supported on 860 parts by weight of a silica/alumina inactive carrier (having a particle size of 3.5 mm) with a 80 wt.% aqueous ethanol solution as a binder.
The thus-obtained supported catalyst was calcined at 300°C for 5 hours to obtain a heteropoly acid catalyst (hereinafter, referred to as catalyst 4). The average particle size of the obtained catalyst was 4.3 mm. The obtained heteropoly acid catalyst had a composition ratio except for oxygen consisting of: 0.6 of vanadium, 1.2 of phosphorus, 0.4 of arsenic and 0.4 of copper, based on 10 of molybdenum.

For measurement of the hot spot temperature, the catalyst 4 was filled in a steel reaction tube having an internal diameter of 29.4 mm which is equipped with a thermocouple protection tube having an external diameter of 6 mm so that the height of the filling layer was 350 cm. As a material gas, a reaction product gas obtained by oxidizing isobutylene with molecular oxygen in the presence of a complex oxide catalyst containing molybdenum, bismuth, cobalt and iron as a main component was used. The composition (molar ratio) of said reaction product gas was comprised of 3.21% of methacrolein, 8.99% of oxygen, 71.54% of nitrogen, 14.46% of water vapor, 0.12% of methacrylic acid and 1.68% of the others, per volume. Said reaction product gas was supplied into the above-described reaction tube so that the space velocity was 800 h⁻¹. After starting the reaction, partial oxidation reaction of methacrolein was continued while adjusting the reaction bath temperature so that the methacrolein conversion ratio was 75% ± 2%. The outlet pressure of the reactor was adjusted to be 0.5 kG (50 kPaG).
When the reaction was continued for 16000 hours, the reaction bath temperature was 297°C, the methacrolein conversion ratio was 77%, the hot spot temperature was 317°C and the methacrylic acid selectivity was 81.5%.
Subsequently, this used catalyst was taken from the reaction tube and the whole volume thereof was recovered. To 2000 g of deionized water, 2000 g of the taken-out catalyst were added, the mixture was stirred for 30 minutes and then filtered by suction using a filter paper. While leaving the filtration residue in the nutsche, 1000 g of deionized water were added and again an operation of filtration by suction was carried out three times in all. All the obtained filtrates were combined to obtain a filtrate (solution A). A solid content finally left was about 1000 g and had silica and alumina as a main component according to the qualitative analysis by fluorescence X-ray analysis.

The weight of the filtrate (solution A) recovered in the above process was measured, the rest after sampling 100 g thereof was transferred to a jacket-type furnace, 5000 g of deionized water were added to the solution A, and then 69 g of IXE-300 manufactured by by Toagosei Co., Ltd. were added to the mixture, which was then stirred for 30 minutes followed by filtration by suction using a filter paper.
The sampled filtrate (solution A) was dried in an evaporator to obtain a powder, which was subjected to quantitative analysis by fluorescence X-ray analysis to find that the components and weights to be added were: 174.3 g of molybdenum trioxide, 3.86 g of vanadium pentaoxide, 0.23 g of a 85 wt.% aqueous phosphoric acid solution and 0.37 g of cupric acetate.
Said solution A was again transferred to the jacket-type furnace and heated with stirring at 90°C for 2 hours in order to remove ethanol. After that, when 900 ml of a 30 wt.% aqueous hydrogen peroxide solution were gradually added while continuing stirring, the dark green solution A turned to an orange yellow transparent solution B. To the solution B, 174.3 g of molybdenum trioxide, 3.86 g of vanadium pentaoxide, 0.23 g of a 85 wt.% aqueous phosphoric acid solution and 0.37 g of cupric acetate were added and the mixture was heated with stirring at 90°C for 90 minutes to obtain a solution C. This solution C was dried with a spray dryer to obtain catalyst granules.
Then, by the same method as that of manufacturing the above-described catalyst 4, a regenerated supported catalyst (catalyst E4) was manufactured.
In the same manner as in Example 1, partial oxidation reaction of methacrolein in E4 was carried out. The results are shown in Table 1.

### Comparative Examples 1

In the same manner as in Example 1 except that molybdenum trioxide, vanadium pentoxide and copper oxide were not additionally added, a molded catalyst E5 was prepared. The partial oxidation reaction results of methacrolein in E5 catalyst are shown in Stable 1.

### Comparative Examples 2

In the same manner as in Example 1 except that a solution B was not prepared and molybdenum trioxide, vanadium pentoxide and copper oxide were not added, a molded catalyst E6 was prepared. The partial oxidation reaction results of methacrolein in E6 catalyst are shown in Stable 1.

**Table 1: Partial oxidation reaction result of methacrolein**

| | | | Methacrolein conversion acid (%) | Methacrylic acid selectivity (%) | Methacrylic acid yield (%) |
|---|---|---|---|---|---|
| Example 1 | catalyst 1 | 3 hours after staring reaction | 78.55 | 79.55 | 62.49 |
| | | After high temperature reaction treatment | 83.11 | 80.39 | 66.81 |
| | catalyst E1 | 3 hours after staring reaction | 78.61 | 79.04 | 62.14 |
| | | After high temperature reaction treatment | 83.22 | 80.36 | 66.88 |
| Example 2 | catalyst 2 | 3 hours after staring reaction | 77.92 | 78.40 | 61.09 |
| | | After high temperature reaction treatment | 87.00 | 76.32 | 66.40 |
| | catalyst E2 | 3 hours after staring reaction | 77.10 | 78.51 | 60.54 |
| | | After high temperature reaction treatment | 86.88 | 76.79 | 66.71 |
| Example 3 | catalyst 3 | 3 hours after staring reaction | 72.10 | 77.25 | 55.70 |
| | | After high temperature reaction treatment | 73.01 | 81.65 | 59.61 |
| | catalyst E3 | 3 hours after staring reaction | 72.43 | 77.27 | 55.97 |
| | | After high temperature reaction treatment | 73.47 | 81.90 | 60.17 |
| Example 4 | catalyst E4 | 3 hours after staring reaction | 78.50 | 79.78 | 62.63 |
| | | After high temperature reaction treatment | 83.77 | 80.06 | 67.07 |
| Comparative Example 1 | catalyst E5 | 3 hours after staring reaction | 68.78 | 83.24 | 57.26 |
| | | After high temperature reaction treatment | 70.92 | 84.78 | 60.12 |
| Comparative Example 2 | catalyst E6 | 3 hours after staring reaction | 59.34 | 81.86 | 48.57 |
| | | After high temperature reaction treatment | 71.62 | 85.79 | 61.44 |

As is clear from the above-described Table 1, the target catalyst and the regenerated catalyst show equivalent results for any of methacrolein conversion ratio, methacrolein selectivity and methacrolein yield after 3 hours and also after high temperature treatment, and it is found that the regenerated catalyst obtained according to the present invention is a catalyst having performance equivalent to an unused target catalyst and having no problems even when used in combination with an unused target catalyst.

### Test Example

For measurement of the hot spot temperature, the catalyst E2 (regenerated supported catalyst) obtained in Example 2 was filled in a steel reaction tube having an internal diameter of 29.4 mm which was equipped with a thermocouple protection tube having an external diameter of 6 mm so that the height of the filling layer was 350 cm. As a material gas, a reaction product gas obtained by oxidizing isobutylene using a molecular oxygen in the presence of a complex oxide catalyst containing molybdenum, bismuth, cobalt and iron as a main component was used. The composition (molar ratio) of said reaction product gas was comprised of 3.21% of methacrolein, 8.99% of oxygen, 71.54% of nitrogen, 14.46% of water vapor, 0.12% of methacrylic acid and 1.68% of the others, per volume. Said reaction product gas was supplied into said reaction tube so that the space velocity was 1000 h⁻¹. After starting the reaction, the partial oxidation reaction of methacrolein was continued while adjusting the reaction bath temperature so that the methacrolein conversion ratio was 75% ± 2%. The outlet pressure of the reactor was adjusted to be 0.5 kG (50 kPaG).
As the results of the methacrolein oxidation reaction 2000 hours after the starting the reaction, the reaction bath temperature was 300°C, the hot spot temperature was 315°C, the methacrolein conversion ratio was 77.5% and the methacrylic acid selectivity was 83.9%.

### Industrial Applicability

Any of the regenerated catalysts obtained by the manufacturing method of the present invention has performance equivalent to an unused target catalyst, poses no problem in use with an unused target catalyst, and also allows a simple manufacturing method and effective utilization of any of a recovered catalyst from manufacturing process and a used catalysts.

## Claims

1. A method for manufacturing a catalyst, which is **characterized by** comprising the following processes for manufacturing a regenerated heteropoly acid-based catalyst using, as a raw material, a recovered heteropoly acid-based catalyst containing molybdenum, phosphorus, vanadium or copper as an essential component:
Process a: a process to prepare a solution A by mixing a heteropoly acid-based catalyst with an aqueous solvent and removing a component insoluble in the solvent;
Process b: a process of measuring the molar quantity of at least one component of molybdenum, phosphorus, vanadium or copper contained in the solution A;
Process c: a process wherein an aqueous hydrogen peroxide solution is added to the solution A or a solution obtained in the below-described Process d to oxidize the heteropoly acid;
Process d: a process wherein the difference between the above molar quantity obtained in Process b) and the mol theoretical value of an element required for preparation of an target regenerated catalyst is determined and the shortage amount of a catalyst component is added to the solution A or the solution obtained in Process c to prepare a solution containing the additional raw material component;
Process e: a process to prepare catalyst granules D by drying the solution obtained through Process a to Process d; and
Process f: a process to prepare a molded catalyst E by molding and subsequently calcining the catalyst granules D.

2. The method for manufacturing a catalyst according to Clam 1, wherein the heteropoly acid-based catalyst further contains one kind or more selected from the below-described X and/or one kind or more selected from Y; and the content of at least one component of molybdenum, phosphorus, vanadium, copper, X or Y is measured in Process b:
X: alkali metal, alkali earth metal or ammonia;
Y: silver, zirconium, arsenic, boron, germanium, tin, lead, chrome, bismuth, cobalt, nickel, cerium, tungsten, iron, aluminum, magnesium, antimony, niobium, manganese or titanium.

3. The method for manufacturing a catalyst according to Claim 1, wherein the above heteropoly acid-based catalyst is a catalyst represented by the below-described general formula:
MoₐP_{b}V_{c}Cu_{d}XₑY_{f}O_{g} (wherein, Mo, P, V and Cu respectively represent molybdenum, phosphorus,
vanadium and copper; X represents at least one kind selected from an alkali metal,
an alkali earth metal and ammonia and Y represents at least one kind selected from the group consisting of silver, zirconium, arsenic, boron, germanium, tin, lead, chrome, bismuth, cobalt, nickel, cerium, tungsten, iron, aluminum, magnesium, antimony, niobium, manganese or titanium, respectively; each symbol of a to g is an atomic ratio of the element, where b is 0.1 to 6, c is 0.1 to 6, d is 0.1 to 4.0, e is 0 to 4, f is 0 to 5 when a = 10, and g is a numerical value determined depending on the oxidation state of each element).

4. The method for manufacturing a catalyst according to Claim 1, wherein the heteropoly acid-based catalyst is a catalyst for manufacturing methacrylic acid by gas-phase partial oxidation reaction of methacrolein.

5. The method for manufacturing a catalyst according to Claim 1, wherein the heteropoly acid-based catalyst is a recovered product of a waste catalyst generated in a process for manufacturing a gas-phase oxidation catalyst for manufacturing methacrylic acid from methacrolein or a recovered product of a product in process of said gas-phase oxidation catalyst.

6. The method for manufacturing a catalyst according to Claim 2, wherein X is cesium and/or ammonia.

7. The method for manufacturing a catalyst according to Claim 2, wherein Y is antimony and/or arsenic.

8. The method for manufacturing a catalyst according to Claim 2, wherein the catalyst is a catalyst not containing X.

9. The method for manufacturing a catalyst according to any one of Claims 3 to 8, wherein e is 0, and Y is at least one element selected from the group consisting of arsenic, antimony and cerium.

10. The method for manufacturing a catalyst according to Claim 1, wherein the molded catalyst E is a molded catalyst where an inactive carrier is coated with the catalyst granules D using a liquid binder.
